# EUROPEAN PATENT SPECIFICATION

(11) **EP 1 713 912 B1**
(45) Date of publication and mention of the grant of the patent: **18.09.2013**
(21) Application number: 05700612.4
(22) Date of filing: 28.01.2005
(51) Int. Cl.: C12N 15/113

(54) **MODIFIED SHORT INTERFERING RNA (MODIFIED SIRNA)**
MODIFIZIERTE SIRNA (SHORT INTERFERING RNA)
ARN A INTERFERENCE BREVE MODIFIES (ARN SB SI /SB MODIFIES)

(30) Priority: 30.01.2004 DK 200400145; 22.03.2004 WO PCT/DK2004/000192; 08.07.2004 DK 200401079
(43) Date of publication of application: 25.10.2006
(73) Proprietor: Santaris Pharma A/S, 2970 Hørsholm (DK)
(72) Inventor: ELMEN, Joacim, 120 67 Stockholm (SE); WAHLESTEDT, Claes, Jupiter, FL 33458 (US); LIANG, Zicai, S-174 46 Sundbyberg (SE); ORUM, Henrik, DK-3500 Vaerlose (DK); KOCH, Troels, DK-2300 K benhavn S (DK)
(74) Representative: Turner, Mark Frederic Paris
(86) International application number: PCT/DK2005/000062
(87) International publication number: WO 2005/073378

(56) References cited:
- WO-A-03/070918
- WO-A-2004/099387
- BRAASCH D.A. ET AL.: "RNA interference in mammalian cells by chemically-modified RNA" BIOCHEMISTRY, vol. 42, no. 26, 2003, pages 7967-7975, XP002328494 cited in the application
- PARRISH S. ET AL.: "Functional anatomy of a dsRNA trigger: differential requirement for the two trigger strands in RNA interference" MOLECULAR CELL, vol. 6, November 2000 (2000-11), pages 1077-1087, XP002328495

## Description

### FIELD OF THE INVENTION

The present invention is directed to novel, modified siRNA which are significantly impaired in their ability to support cleavage of mRNA when incorporated into a RISC complex. Such modified siRNA may be useful as therapeutic agents, e.g., in the treatment of various cancer forms.

### BACKGROUND OF THE INVENTION

Discovery of RNA interference (RNAi) in C. Elegans was made by Fire et al. (Nature, 1998, 391, 806-811). Long stretches of double stranded RNA (dsRNA) was found to have a potent knock-down effect on gene expression that could last for generations in the worm. RNA interference (RNAi) rapidly became a functional genomic tool in C. Elegans (early RNA interference is reviewed by Fire (TIG, 1999,15, 358-363) and Bosher and Labouesse (Nature Cell Biology, 2000, 2, E31-E36)). The first studies where RNA interference was demonstrated to work in vertebrates were performed in zebrafish embryos and mouse oocytes (Wargelius et al., Biochem. Biophys. Res. Com. 1999, 263, 156-161, Wianny and Zernicka-Goetz, Nature Cell Biology, 2000, 2, 70-75). Since dsRNA induces non-specific effects in mammalian cells it has been argued that these mechanisms were not fully developed in the mouse embryonic system (Alexopoulou et al., Nature, 2001, 413, 732-738, Reviews: Stark et al., Annu. Rev. Biochem., 1998, 67, 227-264 and Samuel, Clin. Micro. Rev., 2001, 14, 778-809).

As far as C. Elegans and Drosophila are concerned, it has been shown that the long RNAi strands are degraded to short double strands (21-23 nucleotides) and that these degraded forms mediated the interference (Zamore et al., Cell, 2000, 101, 25-33 and Elbashir et al., Gen. Dev., 2001, 15, 188-200). Elbashir et al. (Gen. Dev., 2001, 15, 188-200) showed that a sense or antisense target is cleaved equally and that both strands in siRNA can guide cleavage to target antisense or sense RNA, respectively. It was unambiguously shown by Elbashir et al. (Nature, 2001, 411, 494-498) that the siRNAs mediate potent knock-down in a variety of mammalian cell lines and probably escaped the adverse non-specific effects of long dsRNA in mammalian cells. This discovery was a hallmark in modern biology and the application of siRNAs as therapeutics soon became an attractive field of research (Reviewed by McManus and Sharp, Nature Reviews Genetics, 2002, 3, 737-747 and Thompson, DDT, 2002, 7, 912-917).

DsRNAs are rather stable in biological media. However, the moment the duplex is dissociated into the individual strands these are, by virtue of being RNA, immediately degraded. One of the strategies to bring further stability to siRNA has been to introduce chemically modified RNA residues into the individual strands of the siRNA. It is well known that synthetic RNA analogues are much more stable in biological media, and that the increased stability is also induced to the proximate native RNA residues. By greater stability is mainly meant increased nuclease resistance but also better cellular uptake and tissue distribution may be conferred by such modifications. Several siRNA analogues have been described:

Pre-siRNA (Parrish et al. Mol. Cell, 2000, 6, 1077-1087) show tolerance for certain backbone modifications for RNAi in C. elegans. By *in vitro* transcription of the two different strands in presence of modified nucleotides, it was possible to show that phosphorothioates are tolerated in both the sense and antisense strand and so are 2'-fluorouracil instead of uracil. 2'-aminouracil and 2'-aminocytidine reduce the RNAi activity when incorporated into the sense strand and the activity is completely abolished when incorporated in the antisense strand. With an exchange of uracil to 2'-deoxythymidine in the sense strand the effect is also reduced, and even more when the exchange is in the antisense strand. If one or both strand(s) consist entirely of DNA monomers, the RNAi activity is also abolished. In the above-mentioned study, base modifications were also investigated; It was found that 4-thiouracil and 5-bromouracil were tolerated in both stands, whereas 5-iodouracil and 5-(3-aminoallyl)uracil reduce the effect in the sense strand and even more in the antisense strand. Replacing guanosine with inosine markedly reduced the activity, independtly of whether the modification was performed in the sense or antisense strand.

However, UU 3' overhangs can be exchanged with 2'deoxythymidine 3' overhangs and are well tolerated (Elbashir et al., Nature, 2001, 411, 494-498 and Boutla et al., Curr. Biol., 2001, 11, 1776-1780).

It has also been shown that DNA monomers can be incorporated in the sense strand without compromising the activity.

Elbashir et al., EMBO, 2001, 20, 6877-6888) showed that modified siRNA containing four deoxynucleotides in each 3'-end of the siRNA maintained full activity. Furthermore, it was found that the activity was abolished if the siRNA contained only one base-pair mismatch in the "middle" of the molecule.

However, it has also been reported that 1-2 mismatches can be tolerated as long as the mismatches are introduced in the sense strand (Holen et al., NAR, 2002, 30, 1757-1766; Hohjoh, FEBS Lett., 2002, 26179, 1-5; Hamada et al., Antisense and Nucl. Acid Drug Dev., 2002, 12, 301-309; and Boutla et al., Curr. Biol., 2001, 11, 1776-1780).

Nykänen et al. (Cell, 2001, 107, 309-321) showed the need for ATP in making siRNA out of RNAi, but also in the later steps to exert the siRNA activity. ATP is needed for unwinding and maintaining a 5'-phosphate for RISC recognition. The 5'-phosphate is necessary for siRNA activity. Martinez et al. (Cell, 2002, 110, 563-574) showed that a single strand can reconstitute the RNA-induced silencing complex (RISC, Hammond et al., Nature, 2000, 404, 293-296) and that a single antisense strand has activity especially when 5'-phosphorylated. 5'-antisense strand modification inhibits activity while both the 3' end and the 5' end of the sense strand can be modified.

Amarzguioui et al. (NAR, 2003, 31, 589-595) confirmed the above-mentioned findings, and it was concluded that a mismatch is tolerated as long as it is not too close to the 5' end of the antisense strand. A mismatch 3-5 nucleotides from the 5' end of the antisense strand markedly diminishes the activity. However, it was shown that two mismatches are tolerated if they are in the "middle" or towards the 3' end of the antisense strand, though with a slightly reduced activity.

Modifications, such as phosphorothioates and 2'-O-methyl RNA, have been introduced at the termini of siRNA (Amarzguioui et al., NAR, 2003, 31, 589-595) and they were well tolerated. 2'-O-allylation reduces the effect when present in the 5' end of the antisense strand

The bi-cyclic nucleoside analogue ENA (2'-O,4'-C-ethylene thymidine (ENA thymidine, eT) has also been incorporated into siRNA (Hamada et al., Antisense and Nucl. Acid Drug Dev., 2002, 12, 301-309). It was shown that two ENA thymidines in the 5' end of the sense strand deteriorated the effect. It was concluded by Hamada *et al.* (2002) that: "*using 2'-O,4'-C-ethylene thymidine, which is a component of ethylene-bridged nucleic acids (ENA), completely abolished RNAi".*

More recently, a number of siRNAs containing incorporated LNA monomers were described by Braasch et al. (Biochemistry 2003, 42, 7967-7975).

In conclusion, it has been shown that the antisense strand is more sensitive to modifications than is the sense strand. Without being limited to any specific theory, this phenomena is, at least partly, believed to be based on the fact that the structure of the antisense/target duplex has to be native A-form RNA. The sense strand of siRNA can be regarded as a "vehicle" for the delivery of the antisense strand to the target and the sense strand is not participating in the enzyme-catalysed degradation of RNA. Thus, in contrast to the antisense strand, modifications in the sense strand is tolerated within a certain window even though the modifications induce changes to the A-form structure of the siRNA. If changes are introduced in the antisense strand they have to be structurally balanced within the recognition frame of the native RNA induced silencing complex (RISC).

### BRIEF DESCRIPTION OF THE INVENTION

As discussed above siRNAs have been shown to be able to potently suppress translation of complementary mRNAs inside cells. The mechanism by which these molecules work have been characterised in some detail. Briefly, after introduction into the cell by transfection one of the two RNA strands of the siRNA gets incorporated into the an enzyme complex (termed RISC), which thereby acquires the ability to bind to and cleave a mRNA containing a complementary sequence (thereby preventing its translation into protein). It has been shown that each of the two strands of the siRNA can be incorporated into the RISC complex, but that the strand that forms the weakest basepair at its 5'-end is preferred. Therefore, any siRNA will - to a different extent - lead to the production of a mixture of activated RISC complexes that can cleave both the intended target as well as non-targets. It is evident that when siRNAs are used as a therapeutic drug it is highly desirable that the vast majority, preferably all, of the activated RISC complex contains the siRNA strand that is complementary to the desired target. Therefore, modified siRNAs that *i*) eliminates incorporation of the non-desired siRNA strand into the RISC complex, and/or *ii*) disables the mRNA's destructive activity of inappropriately incorporated siRNA strands, would be highly desirable as such siRNAs would be expected to exert fewer side-effects than the corresponding non-modified siRNA drug. The present invention is based on the surprising finding that the mRNA-cleaving capability of an activated RISC complex can be suppressed by modifying nucleotides at specific positions in the sense strand.

Accordingly, in a first aspect the present invention relates to a modified siRNA comprising a sense strand and an antisense strand, wherein the sense strand comprises a modified RNA nucleotide in at least position 10, calculated from the 5'-end.

In another aspect the present invention relates to a modified RNA oligomer comprising 12-35 monomers, wherein said oligomer comprises a modified RNA nucleotide in at least position 10, calculated from the 5'-end.

In still another aspect the present invention relates to a pharmaceutical composition comprising a modified siRNA according to the invention and a pharmaceutically acceptable diluent, carrier or adjuvant.

In a further aspect the present invention relates to a modified siRNA according to the invention for use as a medicament.

In a still further aspect the present invention relates to the use of a modified siRNA according to the invention for the manufacture of a medicament for the treatment of cancer, an infectious disease or an inflammatory disease.

In an even further aspect the oligomers of the present invention may be used in a method for treating cancer, an infectious disease or an inflammatory disease, said method comprising administering a modified siRNA according to the invention or a pharmaceutical composition according to the invention to a patient in need thereof.

Other aspects of the present invention will be apparent from the below description and the appended claims.

### BRIEF DESCRIPTION OF THE DRAWINGS

FIG.1 shows the proposed mechanism of RISC loading where the helicase is unwinding the siRNA at its weakest binding end.
FIG. 2 shows the effect of nucleotide modification in the antisense strand. Lines are RNA, circles indicate LNA monomers. The tested compounds were (from left to right): 2nd bar: GL3+/-; 3rd bar: GL3+/2187; 4th bar: GL3+/2789; 5th bar: GL3+/2790; 6th bar: GL3+/2792: 7th bar: GL3+/2793; 8th bar: GL3+/2794; 9th bar: GL3+/2864; 10th bar: GL3+/2865; 11th bar: GL3+/2866; 12th bar: GL3+/2867.
FIG. 3 shows the decreased siLNA efficacy by using a LNA monomer with a bulky nucleobase (T instead of U) at position 10 in the antisense strand (counted from the 5'-end). The tested compounds were: siRNA: GL3+/-; siLNA10T: GL3+/2865; siLNA10U: GL3+/2865-U.

### DETAILED DESCRIPTION OF THE INVENTION

### Definitions

In the present context, "siRNA" or "small interfering RNA" refers to double-stranded RNA molecules from about 12 to about 35 ribonucleotides in length that are named for their ability to specifically interfere with protein expression.

The term "modified siRNA" means that at least one of the ribonucleotides in the siRNA molecule has been modified in its ribose unit, in its nitrogenous base, in its internucleoside linkage group, or combinations thereof.

In the present context the term "nucleotide" means a 2-deoxyribose (DNA) monomer or a ribose (RNA) monomer which is bonded through its number one carbon to a nitrogenous base, such as adenine (A), cytosine (C), thymine (T), guanine (G) or uracil (U), and which is bonded through its number five carbon atom to an internucleoside linkage group (as defined below) or to a terminal group (as defined below).

Accordingly, when used herein the term "RNA nucleotide" or "ribonucleotide" encompasses a RNA monomer comprising a ribose unit which is bonded through its number one carbon to a nitrogenous base selected from the group consisting of A, C, G and U, and which is bonded through its number five carbon atom to a phosphate group or to a terminal group.

Analogously, the term "DNA nucleotide" or "2-deoxyribonucleotide" encompasses a DNA monomer comprising a 2-deoxyribose unit which is bonded through its number one carbon to a nitrogenous base selected from the group consisting of A, C, T and G, and which is bonded through its number five carbon atom to a phosphate group or to a terminal group.

When used herein the term "modified RNA nucleotide" or "modified ribonucleotide" means that the RNA nucleotide in question has been modified in its ribose unit, in its nitrogenous base, in its internucleoside linkage group, or combinations thereof. Accordingly, a "modified RNA nucleotide" may contain a sugar moiety which differs from ribose, such as a ribose monomer where the 2'-OH group has been modified. Alternatively, or in addition to being modified at its ribose unit, a "modified RNA nucleotide" may contain a nitrogenous base which differs from A, C, G and U (a "non-RNA nucleobase"), such as T or ^{Me}C. Finally, a "modified RNA nucleotide" may contain an internucleoside linkage group which is different from phosphate (-O-P(O)₂-O-), such as phosphorothioate (-O-P(O,S)-O-).

The term "DNA nucleobase" covers the following nitrogenous bases: A, C, T and G.

The term "RNA nucleobase" covers the following nitrogenous bases: A, C, U and G.

As used herein, the "non-RNA nucleobase" encompasses nitrogenous bases which differ from A, C, G and U, such as purines (different from A and G) and pyrimidines (different from C and U).

In the present context, the term "nucleobase" covers DNA nucleobases, RNA-nucleobases and non-RNA nucleobases.

When used herein the term "sugar moiety which differs from ribose" refers to a pentose with a chemical structure that is different from ribose. Specific examples of sugar moieties which are different from ribose include ribose monomers where the 2'-OH group has been modified.

When used in the present context, the terms "locked nucleic acid monomer", "locked nucleic acid residue", "LNA monomer" or "LNA residue" refer to a bicyclic nucleotide analogue. LNA monomers are described in *inter alia* WO 99/14226, WO 00/56746, WO 00/56748, WO 01/25248, WO 02/28875, WO 03/006475 and WO 03/095467. The LNA monomer may also be defined with respect to its chemical formula. Thus, a "LNA monomer" as used herein has the chemical structure shown in Scheme 1 below: 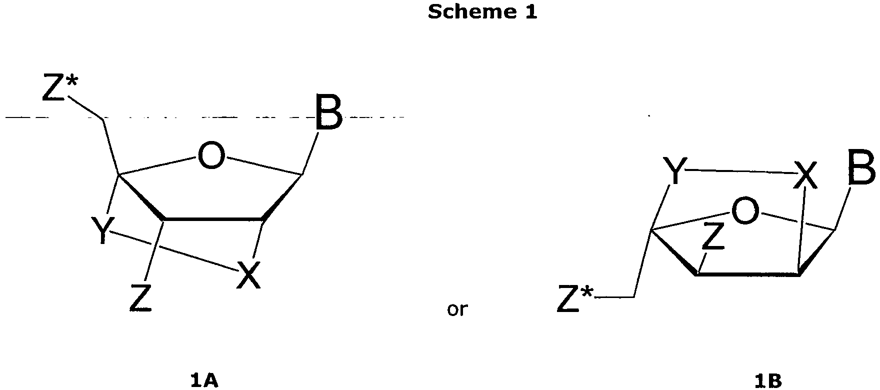 wherein
X is selected from the group consisting of O, S and NR^{H}, where R^{H} is H or alkyl, such as C₁₋₄-alkyl;
Y is (-CH₂)ᵣ, where r is an integer of 1-4;
Z and Z* are independently absent or selected from the group consisting of an internucleoside linkage group, a terminal group and a protection group; and
B is a nucleobase.

The term "internucleoside linkage group" is intended to mean a group capable of covalently coupling together two nucleosides, two LNA monomers, a nucleoside and a LNA monomer, etc. Specific and preferred examples include phosphate groups and phosphorothioate groups.

The term "nucleic acid" is defined as a molecule formed by covalent linkage of two or more nucleotides. The terms "nucleic acid" and "polynucleotide" are used interchangeable herein. When used herein, a "nucleic acid" or a "polynucleotide" typically contains more than 35 nucleotides.

The term "oligonucleotide" refers, in the context of the present invention, to an oligomer (also called oligo) of RNA, DNA and/or LNA monomers as well as variants and analogues thereof. When used herein, an "oligonucleotide" typically contains 2-35 nucleotides, in particular 12-35 nucleotides.

The terms "unit", "residue" and "monomer" are used interchangeably herein.

The term "at least one" encompasses an integer larger than or equal to 1, such as 1, 2, 3, 4, 5, 6, 7, 8, 9, 10, 11, 12, 13, 14, 15, 16, 17 and so forth.

The terms "a" and "an" as used about a nucleotide, an active agent, a LNA monomer, etc. is intended to mean one or more. In particular, the expression "a component (such as a nucleotide, an active agent, a LNA monomer or the like) selected from the group consisting of ..." is intended to mean that one or more of the cited components may be selected. Thus, expressions like "a component selected from the group consisting of A, B and C" is intended to include all combinations of A, B and C, i.e. A, B, C, A+B, A+C, B+C and A+B+C.

The term "thio-LNA" refers to a locked nucleotide in which X in Scheme 1 is S. Thio-LNA can be in both the beta-D form and in the alpha-L form. Generally, the beta-D form of thio-LNA is preferred. The beta-D form of thio-LNA is shown in Scheme 2 as compound **2C.**

The term "amino-LNA" refers to a locked nucleotide in which X in Scheme 1 is NH or NR^{H}, where R^{H} is hydrogen or C₁₋₄-alkyl. Amino-LNA can be in both the beta-D form and alpha-L form. Generally, the beta-D form of amino-LNA is preferred. The beta-D form of amino-LNA is shown in Scheme 2 as compound **2D.**

The term "oxy-LNA" refers to a locked nucleotide in which X in Scheme 1 is O. Oxy-LNA can be in both the beta-D form and alpha-L form. The beta-D form of oxy-LNA is preferred. The beta-D form and the alpha-L form are shown in Schemes 2 and 3 as compounds **2A** and **2B,** respectively.

As used herein, the term "mRNA" means the mRNA transcript(s) of a targeted gene, and any further transcripts, which may be identified.

As used herein, the term "target nucleic acid" encompass any RNA that would be subject to modulation, targeted cleavage, steric blockage (decrease the abundance of the target RNA and/or inhibit translation) guided by the antisense strand. The target RNA could, for example, be genomic RNA, genomic viral RNA, mRNA or a pre-mRNA

As used herein, the term "target-specific nucleic acid modification" means any modification to a target nucleic acid.

As used herein, the term "gene" means the gene including exons, introns, non-coding 5' and 3' regions and regulatory elements and all currently known variants thereof and any further variants, which may be elucidated.

As used herein, the term "modulation" means either an increase (stimulation) or a decrease (inhibition) in the expression of a gene. In the present invention, inhibition is the preferred form of modulation of gene expression and mRNA is a preferred target.

As used herein, the term "targeting" an siLNA or siRNA compound to a particular target nucleic acid means providing the siRNA or siLNA oligonucleotide to the cell, animal or human in such a way that the siLNA or siRNA compounds are able to bind to and modulate the function of the target.

As used herein, "hybridisation" means hydrogen bonding, which may be Watson-Crick, Hoogsteen, reversed Hoogsteen hydrogen bonding, etc., between complementary nucleoside or nucleotide bases. The four nucleobases commonly found in DNA are G, A, T and C of which G pairs with C, and A pairs with T. In RNA T is replaced with uracil (U), which then pairs with A. The chemical groups in the nucleobases that participate in standard duplex formation constitute the Watson-Crick face. Hoogsteen showed a couple of years later that the purine nucleobases (G and A) in addition to their Watson-Crick face have a Hoogsteen face that can be recognised from the outside of a duplex, and used to bind pyrimidine oligonucleotides via hydrogen bonding, thereby forming a triple helix structure.

In the context of the present invention "complementary" refers to the capacity for precise pairing between two nucleotides sequences with one another. For example, if a nucleotide at a certain position of an oligonucleotide is capable of hydrogen bonding with a nucleotide at the corresponding position of a DNA or RNA molecule, then the oligonucleotide and the DNA or RNA are considered to be complementary to each other at that position. The DNA or RNA strand are considered complementary to each other when a sufficient number of nucleotides in the oligonucleotide can form hydrogen bonds with corresponding nucleotides in the target DNA or RNA to enable the formation of a stable complex. To be stable *in vitro* or *in vivo* the sequence of a siLNA or siRNA compound need not be 100% complementary to its target nucleic acid. The terms "complementary" and "specifically hybridisable" thus imply that the siLNA or siRNA compound binds sufficiently strong and specific to the target molecule to provide the desired interference with the normal function of the target whilst leaving the function of non-target mRNAs unaffected

In the present context the term "conjugate" is intended to indicate a heterogenous molecule formed by the covalent attachment of a compound as described herein to one or more non-nucleotide or non-polynucleotide moieties. Examples of non-nucleotide or non-polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol (PEG).

In the present context, the term "C₁₋₆-alkyl" is intended to mean a linear or branched saturated hydrocarbon chain wherein the longest chains has from one to six carbon atoms, such as methyl, ethyl, n-propyl, isopropyl, n-butyl, isobutyl, sec-butyl, tert-butyl, pentyl, isopentyl, neopentyl and hexyl. A branched hydrocarbon chain is intended to mean a C₁₋₆-alkyl substituted at any carbon with a hydrocarbon chain.

In the present context, the term "C₁₋₄-alkyl" is intended to mean a linear or branched saturated hydrocarbon chain wherein the longest chains has from one to four carbon atoms, such as methyl, ethyl, n-propyl, isopropyl, n-butyl, isobutyl, sec-butyl and tert-butyl. A branched hydrocarbon chain is intended to mean a C₁₋₄-alkyl substituted at any carbon with a hydrocarbon chain.

When used herein the term "C₁₋₆-alkoxy" is intended to mean C₁₋₆-alkyl-oxy, such as methoxy, ethoxy, n-propoxy, isopropoxy, n-butoxy, isobutoxy, sec-butoxy, tert-butoxy, pentoxy, isopentoxy, neopentoxy and hexoxy.

In the present context, the term "C₂₋₆-alkenyl" is intended to mean a linear or branched hydrocarbon group having from two to six carbon atoms and containing one or more double bonds. Illustrative examples of C₂₋₆-alkenyl groups include allyl, homo-allyl, vinyl, crotyl, butenyl, butadienyl, pentenyl, pentadienyl, hexenyl and hexadienyl. The position of the unsaturation (the double bond) may be at any position along the carbon chain.

In the present context the term "C₂₋₆-alkynyl" is intended to mean linear or branched hydrocarbon groups containing from two to six carbon atoms and containing one or more triple bonds. Illustrative examples of C₂₋₆-alkynyl groups include acetylene, propynyl, butynyl, pentynyl and hexynyl. The position of unsaturation (the triple bond) may be at any position along the carbon chain. More than one bond may be unsaturated such that the "C₂₋₆-alkynyl" is a di-yne or enedi-yne as is known to the person skilled in the art.

The term "carcinoma" is intended to indicate a malignant tumor of epithelial origin. Epithelial tissue covers or lines the body surfaces inside and outside the body. Examples of epithelial tissue are the skin and the mucosa and serosa that line the body cavities and internal organs, such as intestines, urinary bladder, uterus, etc. Epithelial tissue may also extend into deeper tissue layers to from glands, such as mucus-secreting glands.

The term "sarcoma" is intended to indicate a malignant tumor growing from connective tissue, such as cartilage, fat, muscles, tendons and bones.

The term "glioma", when used herein, is intended to cover a malignant tumor originating from glial cells.

### Compounds of the Invention

While LNA monomers can be used freely in the design of modified siLNAs at both 3'-overhangs and at the 5'-end of the sense strand with full activation of the siLNA effect and down-regulation of protein production, the present inventors have surprisingly found that the mRNA-cleaving capability of an activated RISC complex can be suppressed by modifying the sense strand of a siRNA in certain specific positions.

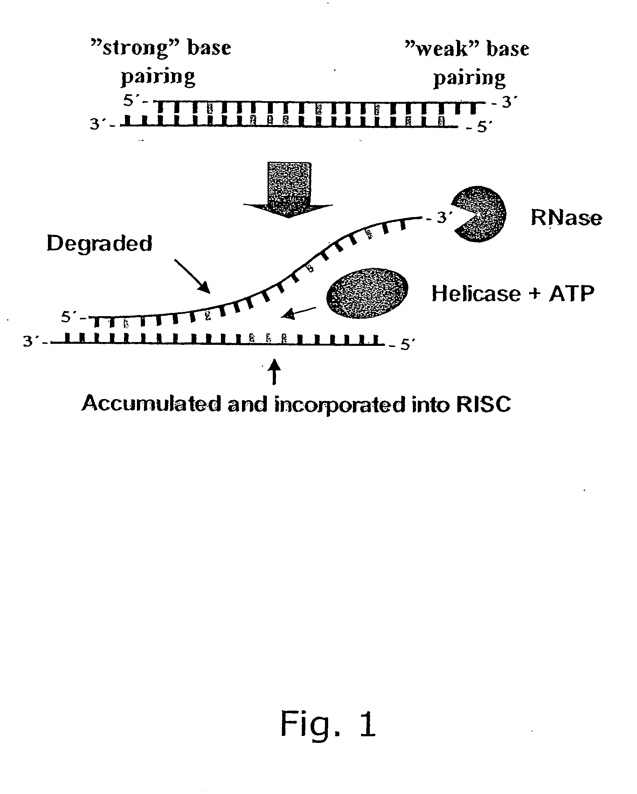

If the LNA monomers are incorporated in the siRNA in such a way that they are strengthening the basepairs in the duplex at the 5'-end of the sense strand, the helicase can thereby be directed to unwinding from the other 5'-end (antisense strand 5'-end). In this way the incorporation of the antisense/guiding strand into RISC can be controlled. The helicase starts unwinding the siRNA duplex at the weakest binding end. The released 3'-end is probably targeted for degradation while the remaining strand is incorporated in the RISC. Efficient siRNAs show accumulation of the antisense/guiding strand and weaker base pairing in the 5'-end of the antisense/guiding strand. Unwanted side effects may possibly be avoided by having only the correct strand (the antisense/guiding strand) in RISC and not the unwanted sense strand (not complementary to the desired target RNA). This mechanism is illustrated in Fig. 1.

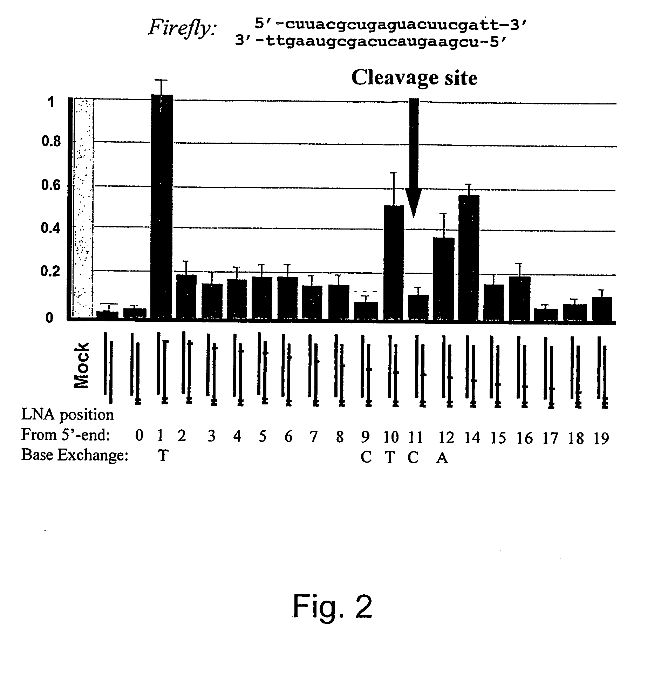

As shown in Fig. 2, the modification RNA-A → LNA-A at position 12 of the antisense strand, counted from the 5'-end, significantly impaired the ability of the siRNA to cleave its target mRNA. In this case, the only difference is the LNA modification in position 12. This is a clear Indication that modification of the position 12 backbone (i.e. the sugar moiety and/or the Internucleoside linkage group) can be used to impair the ability of inappropriately loaded RISC complex to cleave a target mRNA.

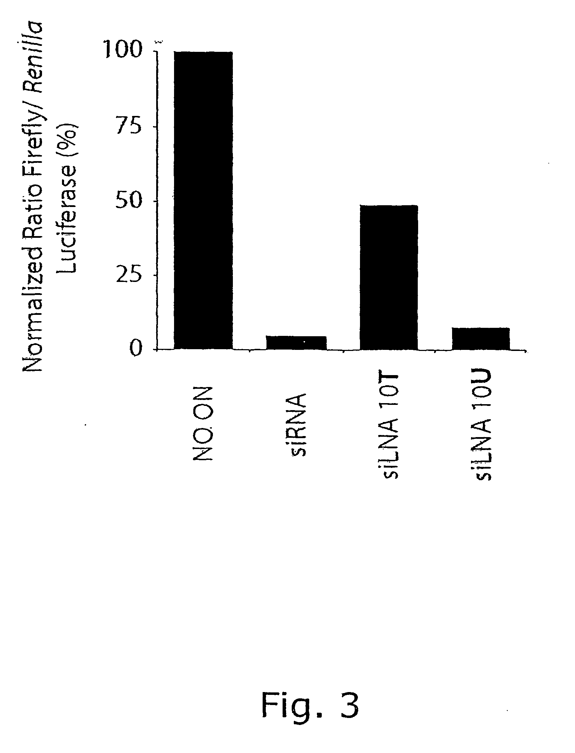

From the data disclosed in Fig. 2 it is also evident that an even more pronounced destructive effect on cleavage can be obtained when the RNA-U at position 10 of the antisense strand, calculated from the 5'-end, is replaced with LNA-T. This indicates that there are several sensitive positions along an siRNA where a single modification of the RNA nucleotide can impair the cleavage activity. Interestingly, the modification RNA-U → LNA-U at position 10 of the antisense strand, counted from the 5'-end, did not result in any measurable reduction in clevage activity (see Fig. 3). This indicates that the substantial activity decrease observed with the LNA-T substitution is mainly caused by the non-RNA nature of the nucleobase rather than by the LNA sugar modification.

These basic observations that gene silencing is reduced when modifications of the position 10 nucleotide of the antisense strand are performed, can be used in the reversed scenario. If the RISC complex should incorporate part of the sense strand and thereby lead to unwanted off-target effects the potency of the unwanted incorporation could be reduced by modifying the sense strand at position 10 in the sense strand.

Accordingly, in its broadest aspect the present invention relates to a modified siRNA which comprises a sense strand and an antisense strand, wherein the sense strand comprises a modified RNA nucleotide in at least positions 10 and optionally at least one position selected from the group consisting of position 11, and position 12, calculated from the 5'-end.

### Modification of the nucleobase

In an interesting embodiment of the invention, the RNA nucleotide is modified in its base structure, i.e. the modified RNA nucleotide comprises a non-RNA nucleobase. In a preferred embodiment of the invention, the sense strand comprises a non-RNA nucleobase in at least one position selected from the group consisting of position 9, position 10, position 11, position 12 and position 13, calculated from the 5'-end. More preferably, the sense strand comprises a non-RNA nucleobase in a position selected from the group consisting of position 10, position 11 and both of positions 10 and 11, calculated from the 5'-end. Most preferably, the sense strand comprises a non-RNA nucleobase in position 10, calculated from the 5'-end.

The non-RNA nucleobase may be any purine or pyrimidine which is different from adenine (A), cytosine (C), uracil (U) and guanine (G). Specific examples of such non-RNA nucleobases include thymine (T), 5-methylcytosine (^{Me}C), isocytosine, pseudoisocytosine, 5-bromouracil, 5-propynyluracil, 5-propyny-6-fluoroluracil, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine, in particular T or ^{Me}C. It will be understood that the actual selection of the non-RNA nucleobase will depend on the corresponding (or matching) nucleotide present in the antisense strand. For example, in case the corresponding antisense nucleotide is A it will normally be necessary to select a non-RNA nucleotide which is capable of establishing hydrogen bonds to A. In this specific case, where the corresponding antisense nucleotide is A, a typical example of a preferred non-RNA nucleobase is T. In a similar way, if the corresponding antisense nucleotide is G, a typical example of a preferred non-RNA nucleobase is ^{Me}C.

### Modification of the sugar moiety

In another interesting embodiment of the invention, the RNA nucleotide is modified in its sugar moiety, i.e. the modified RNA nucleotide comprises a sugar moiety which differs from ribose. In a preferred embodiment of the invention, the sense strand comprises a sugar moiety which differs from ribose, in at least one position selected from the group consisting of position 9, position 10, position 11, position 12 and position 13, calculated from the 5'-end. More preferably, the sense strand comprises a sugar moiety which differs from ribose, in at least one position selected from the group consisting of position 11, position 12 and position 13, calculated from the 5'-end. Most preferably, the sense strand comprises a sugar moiety which differs from ribose in position 12, calculated from the 5'-end.

Preferably, the sugar moiety which differs from ribose is modified in its 2'-OH group. In one embodiment of the invention, the ribose 2'-OH group has been substituted with a group selected from the group consisting of -H, -O-CH₃, -O-CH₂-CH₂-O-CH₃, -O-CH₂-CH₂-CH₂-NH₂, -O-CH₂-CH₂-CH₂-OH and -F, in particular -H. In another, and particular preferred embodiment of the invention, the sugar moiety which differs from ribose is LNA.

The LNA may be selected from the group consisting of thio-LNA, amino-LNA, oxy-LNA and ena-LNA. These LNAs have the general chemical structure shown in Scheme 1 below: 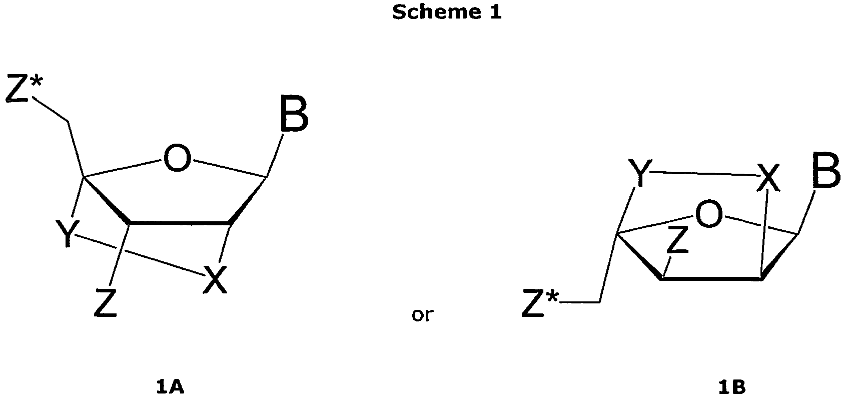 wherein
X is selected from the group consisting of O, S and NR^{H}, where R^{H} is H or alkyl, such as C₁₋₄-alkyl;
Y is (-CH₂)ᵣ, where r is an integer of 1-4
Z and Z* are independently absent or selected from the group consisting of an internucleoside linkage group, a terminal group and a protection group; and
B is a nucleobase.

In a preferred embodiment of the invention, r is 1, i.e. a preferred LNA monomer has the chemical structure shown in Scheme 2 below: 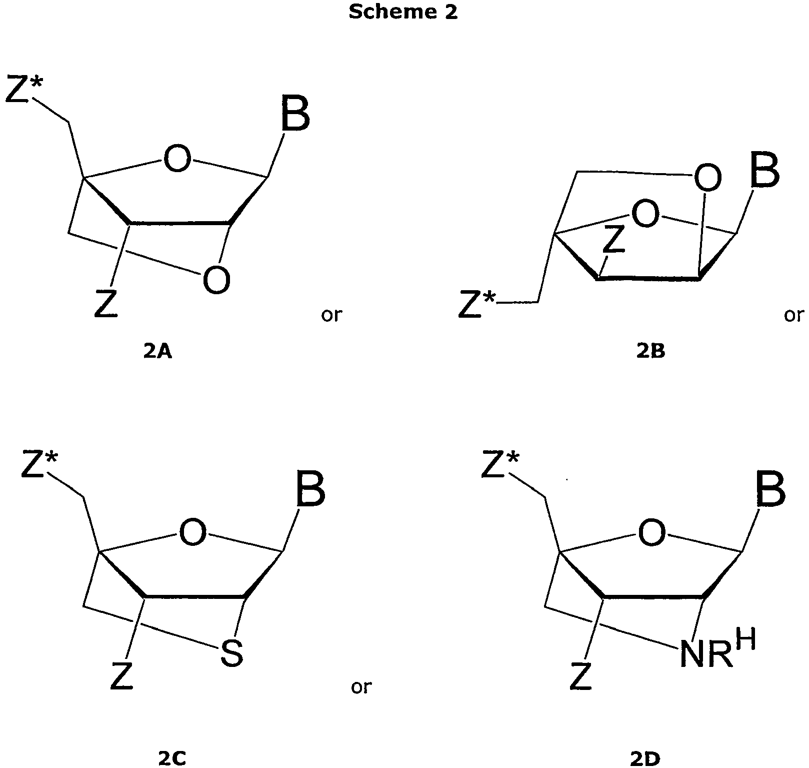 wherein Z, Z*, R^{H} and B are defined above.

In an even more preferred embodiment of the invention, X is O and r is 1, i.e. an even more preferred LNA monomer has the chemical structure shown in Scheme 3 below: 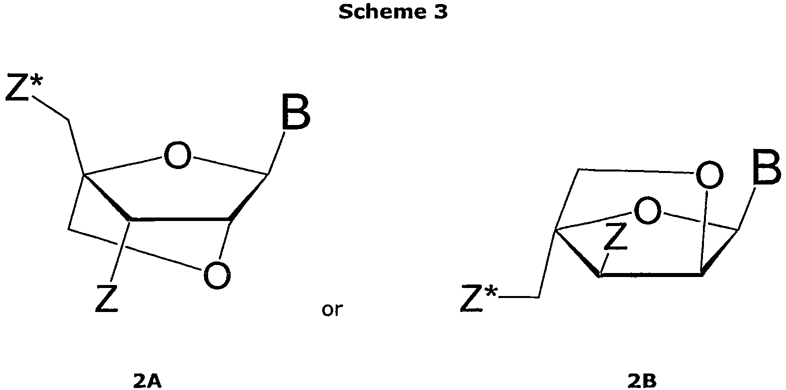 wherein Z, Z* and B are defined above.

The structures shown in **2A** and **2B** above may also be referred to as the "beta-D form" and the "alpha-L form", respectively. In a highly preferred embodiment of the invention, the LNA monomer is the beta-D form, i.e. the LNA monomer has the chemical structure indicated in **2A** above.

As indicated above, Z and Z*, which serve for an internucleoside linkage, are independently absent or selected from the group consisting of an internucleoside linkage group, a terminal group and a protection group depending on the actual position of the LNA monomer within the compound. It will be understood that in embodiments where the LNA monomer is located at the 3' end, Z is a terminal group and Z* is an internucleoside linkage. In embodiments where the LNA monomer is located at the 5' end, Z is absent and Z* is a terminal group. In embodiments where the LNA monomer is located within the nucleotide sequence, Z is absent and Z* is an internucleoside linkage group.

Specific examples of internucleoside linkage groups include -O-P(O)₂-O-, -O-P(O,S)-O-,-O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, -NR^{H}-CO-NR^{H}-, -O-COO-, -O-CO-NR^{H}-, -NR^{H}-CO-CH₂-, -O-CH₂-CO-NR^{H}-, -O-CH₂-CH₂-NR^{H}-, -CO-NR^{H}-CH₂-, -CH₂-NR^{H}-CO-, -O-CH₂-CH₂-S-, -S-CH₂-CH₂-O-, -S-CH₂-CH₂-S-, -CH₂-SO₂-CH₂-, -CH₂-CO-NR^{H}-, - O-CH₂-CH₂-NR^{H}-CO-, -CH₂-NCH₃-O-CH₂-, where R^{H} is hydrogen or C₁₋₄-alkyl.

In a preferred embodiment of the invention, the internucleoside linkage group is a phosphate group (-O-P(O)₂-O-), a phosphorothioate group (-O-P(O,S)-O-) or the compound may contain both phosphate groups and phosphorothioate groups.

Specific examples of terminal groups include terminal groups selected from the group consisting of hydrogen, azido, halogen, cyano, nitro, hydroxy, Prot-O-, Act-O-, mercapto, Prot-S-, Act-S-, C₁₋₆-alkylthio, amino, Prot-N(R^{H})-, Act-N(R^{H})-, mono- or di(C₁₋₆-alkyl)amino, optionally substituted C₁₋₆-alkoxy, optionally substituted C₁₋₆-alk optionally substituted C₂₋₆-alkenyl, optionally substituted C₂₋₆-alkenyloxy, optionally substituted C₂₋₆-alkynyl, optionally substituted C₂₋₆-alkynyloxy, monophosphate including protected monophosphate, monothiophosphate including protected monothiophosphate, diphosphate including protected diphosphate, dithiophosphate including protected dithiophosphate, triphosphate including protected triphosphate, trithiophosphate including protected trithiophosphate, where Prot is a protection group for -OH, -SH and -NH(R^{H}), and Act is an activation group for -OH, -SH, and -NH(R^{H}), and R^{H} is hydrogen or C₁₋₆-alkyl.

Examples of phosphate protection groups include S-acetylthioethyl (SATE) and S-pivaloylthioethyl (t-butyl-SATE).

Still further examples of terminal groups include DNA intercalators, photochemically active groups, thermochemically active groups, chelating groups, reporter groups, ligands, carboxy, sulphono, hydroxymethyl, Prot-O-CH₂-, Act-O-CH₂-, aminomethyl, Prot-N(R^{H})-CH₂-, Act-N(R^{H})-CH₂-, carboxymethyl, sulphonomethyl, where Prot is a protection group for -OH, -SH and -NH(R^{H}), and Act is an activation group for -OH, -SH, and -NH(R^{H}), and R^{H} is hydrogen or C₁₋₆-alkyl.

Examples of protection groups for -OH and -SH groups include substituted trityl, such as 4,4'-dimethoxytrityloxy (DMT), 4-monomethoxytrityloxy (MMT); trityloxy, optionally substituted 9-(9-phenyl)xanthenyloxy (pixyl), optionally substituted methoxytetrahydro-pyranyloxy (mthp); silyloxy, such as trimethylsilyloxy (TMS), triisopropylsilyloxy (TIPS), *tert*-butyldimethylsilyloxy (TBDMS), triethylsilyloxy, phenyldimethylsilyloxy; *tert-*butylethers; acetals (including two hydroxy groups); acyloxy, such as acetyl or halogen-substituted acetyls, *e.g*. chloroacetyloxy or fluoroacetyloxy, isobutyryloxy, pivaloyloxy, benzoyloxy and substituted benzoyls, methoxymethyloxy (MOM), benzyl ethers or substituted benzyl ethers such as 2,6-dichlorobenzyloxy (2,6-Cl₂Bzl). Moreover, when Z or Z* is hydroxyl they may be protected by attachment to a solid support, optionally through a linker.

Examples of amine protection groups include fluorenylmethoxycarbonylamino (Fmoc), *tert-*butyloxycarbonylamino (BOC), trifluoroacetylamino, allyloxycarbonylamino (alloc, AOC), Z-benzyloxycarbonylamino (Cbz), substituted benzyloxycarbonylamino, such as 2-chloro benzyloxycarbonylamino (2-ClZ), monomethoxytritylamino (MMT), dimethoxytritylamino (DMT), phthaloylamino, and 9-(9-phenyl)xanthenylamino (pixyl).

The activation group preferably mediates couplings to other residues and/or nucleotide monomers and after the coupling has been completed the activation group is typically converted to an internucleoside linkage. Examples of such activation groups include optionally substituted O-phosphoramidite, optionally substituted O-phosphortriester, optionally substituted O-phosphordiester, optionally substituted H-phosphonate, and optionally substituted O-phosphonate. In the present context, the term "phosphoramidite" means a group of the formula -P(OR^{x})-N(R^{y})₂, wherein R^{x} designates an optionally substituted alkyl group, *e.g.* methyl, 2-cyanoethyl, or benzyl, and each of R^{y} designates optionally substituted alkyl groups, *e.g*. ethyl or isopropyl, or the group -N(R^{y})₂ forms a morpholino group (-N(CH₂CH₂)₂O). R^{x} preferably designates 2-cyanoethyl and the two R^{y} are preferably identical and designates isopropyl. Accordingly, a particularly preferred phosphoramidite is N,N-diisopropyl-*O*-(2-cyanoethyl)phosphoramidite.

As indicated above, B is a nucleobase which may be of natural or non-natural origin. Specific examples of nucleobases include adenine (A), cytosine (C), 5-methylcytosine (^{Me}C), isocytosine, pseudoisocytosine, guanine (G), thymine (T), uracil (U), 5-bromouracil, 5-propynyluracil, 5-propyny-6, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine.

### Modification of the internucleoside linkage group

In a further interesting embodiment of the invention, the RNA nucleotide is modified in its internucleoside linkage group structure, i.e. the modified RNA nucleotide comprises an internucleoside linkage group which differs from phosphate. In a preferred embodiment of the invention, the sense strand comprises an internucleoside linkage group which differs from phosphate, in at least one position selected from the group consisting of position 9, position 10, position 11, position 12 and position 13, calculated from the 5'-end. More preferably, the sense strand comprises an internucleoside linkage group which differs from phosphate, in at least one position selected from the group consisting of position 11, position 12 and position 13, calculated from the 5'-end. Most preferably, the sense strand comprises an internucleoside linkage group which differs from phosphate in position 12, calculated from the 5'-end.

Herein, the term "the sense strand comprises an internucleoside linkage group which differs from phosphate in position X, calculated from the 5'-end" should be understood so that the internucleoside linkage group establishes a linkage between the 3'-position of the residue in position X and the 5'-position of the residue in position X+1, calculated from the 5'-end.

Specific examples of internucleoside linkage groups which differ from phosphate (-O-P(O)₂-O-) include -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-,-O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, -NR^{H}-CO-NR^{H}-, -O-CO-O-, -O-CO-NR^{H}-, -NR^{H}-CO-CH₂-, -O-CH₂-CO-NR^{H}-, - O-CH₂-CH₂-NR^{H}-, -CO-NR^{H}-CH₂-, -CH₂-NR^{H}-CO-, -O-CH₂-CH₂-S-, -S-CH₂-CH₂-O-, -S-CH₂-CH₂-S-, -CH₂-SO₂-CH₂-, -CH₂-CO-NR^{H}-, -O-CH₂-CH₂-NR^{H}-CO -, -CH₂-NCH₃-O-CH₂-, where R^{H} is hydrogen or C₁₋₄-alkyl. When the internucleside linkage group is modified, the internucleoside linkage group is preferably a phosphorothioate group (-O-P(O,S)-O-).

### Combined and further modifications

As will be understood by the skilled person, any of the above-mentioned modifications may be combined and/or the modified siRNA may contain other modifications which serve the purpose of increasing the biostability (corresponding to an increased Tₘ), increasing the nuclease resistance, improving the cellular uptake and/or improving the tissue distribution.

Therefore, in a highly interesting embodiment of the invention, at least one of the strands of the modified siRNA further comprises at least one modified RNA nucleotide. This further modification may be a modification selected from the group consisting of a non-RNA nucleobase, a sugar moiety which differs from ribose, an internucleoside linkage group which differs from phosphate, and combinations thereof. As will be understood, selectioon of preferred non-RNA nucleobases, preferred sugar moieties which differ from ribose, and preferred internucleotide linkage groups which differ from phosphate will be the same as those described in the sections entitled *"Modification of the nucleobase", "Modification of the sugar moiety"* and *"Modification of the internucleoside linkage group".* For example, in one embodiment of the invention, the sense strand comprises at least one LNA monomer, such as 1-10 LNA monomers, e.g. 1-5 or 1-3 LNA monomers. In another embodiment of the invention, the antisense strand comprises at least one LNA monomer, such as 1-10 LNA monomers, e.g. 1-5 or 1-3 LNA monomers. In a further embodiment of the invention, the sense strand comprises at least one LNA monomer and the antisense strand comprises at least one LNA monomer. For example, the sense strand typically comprises 1-10 LNA monomers, such as 1-5 or 1-3 LNA monomers, and the antisense strand typically comprises 1-10 LNA monomers, such as 1-5 or 1-3 LNA monomers.

In a particular interesting embodiment of the invention the sense strand comprises a sugar moiety which differs from ribose in position 12, calculated from the 5'-end, and a non-RNA nucleobase in position 10, calculated from the 5'-end.

It is known that LNA monomers incorporated into oligos will induce a RNA-like structure of the oligo and the hybrid that it may form. It has also been shown that LNA residues modify the structure of DNA residues, in particular when the LNA residues is incorporated in the proximity of 3'-end. LNA monomer incorporation towards the 5'-end seems to have a smaller effect. This means that it is possible to modify RNA strands which contain DNA monomers, and if one or more LNA residues flank the DNA monomers they too will attain a RNA-like structure. Therefore, DNA and LNA monomers can replace RNA monomers and still the oligo will attain an overall RNA-like structure. As DNA monomers are considerably cheaper than RNA monomers, easier to synthesise and more stable towards nucleolytic degradation, such modifications will therefore improve the overall use and applicability of siRNAs.

Accordingly, in one embodiment at least one (such as one) LNA monomer is located at the 5'-end of the sense strand. Preferably, at least two (such as two) LNA monomers are located at the 5'-end of the sense strand.

In a preferred embodiment of the invention, the sense strand comprises at least one (such as one) LNA monomer located at the 3'-end of the sense strand. More preferably, at least two (such as two) LNA monomers are located at the 3'-end of the of the sense strand.

In a particular preferred embodiment of the invention, the sense strand comprises at least one (such as one) LNA monomer located at the 5'-end of the sense strand and at least one (such as one) LNA monomer located at the 3'-end of the sense strand. Even more preferably, the sense strand comprises at least two (such as two) LNA monomers located at the 5'-end of the sense strand and at least two (such as two) LNA monomers located at the 3'-of the sense strand.

It is preferred that at least one (such as one) LNA monomer is located at the 3'-end of the antisense strand. More preferably, at least two (such as two) LNA monomers are located at the 3'-end of the antisense strand. Even more preferably, at least three (such as three) LNA monomers are located at the 3'-end of the antisense strand. In a particular preferred embodiment of the invention, no LNA monomer is located at or near (i.e. within 1, 2, or 3 nucleotides) the 5'-end of the antisense strand.

In a highly preferred embodiment of the invention, the sense strand comprises at least one LNA monomer at the 5'-end and at least one LNA monomer at the 3'-end, and the antisense strand comprises at least one LNA monomer at the 3'-end. More preferably, the sense strand comprises at least one LNA monomer at the 5'-end and at least one LNA monomer at the 3'-end, and the antisense strand comprises at least two LNA monomers at the 3'-end. Even more preferably, the sense strand comprises at least two LNA monomers at the 5'-end and at least two LNA monomers at the 3'-end, and the antisense strand comprises at least two LNA monomers at the 3'-end. Still more preferably, the sense strand comprises at least two LNA monomers at the 5'-end and at least two LNA monomers at the 3'-end, and the antisense strand comprises at least three LNA monomers at the 3'-end. It will be understood that in the most preferred embodiment, none of the above-mentioned compounds contain a LNA monomer which is located at the 5'-end of the antisense strand.

In a further interesting embodiment of the invention, the LNA monomer is located close to the 3'-end, i.e. at postion 2, 3 or 4, preferably at position 2 or 3, in particular at position 2, calculated from the 3'-end.

Accordingly, in a further very interesting embodiment of the invention, the sense strand comprises a LNA monomer located at position 2, calculated from the 3'-end. In another embodiment, the sense strand comprises LNA monomers located at position 2 and 3, calculated from the 3'-end.

In a particular preferred embodiment of the invention, the sense strand comprises at least one (such as one) LNA monomer located at the 5'-end and a LNA monomer located at position 2 (calculated from the 3'-end). In a further embodiment, the sense strand comprises at least two (such as two) LNA monomers located at the 5'-end of the sense strand a LNA monomer located at positions 2 (calculated from the 3' end).

Furthermore, it is preferred that the antisense strand comprises a LNA monomer at position 2, calculated from the 3'-end. More preferably, the antisense strand comprises LNA monomers in position 2 and 3, calculated from the 3'-end. Even more preferably, the antisense strand comprises LNA monomers located at position 2, 3 and 4, calculated from the 3'-end. In a particular preferred embodiment of the invention, no LNA monomer is located at or near (i.e. within 1, 2, or 3 nucleotides) the 5'-end of the antisense strand.

In a highly preferred embodiment of the invention, the sense strand comprises at least one LNA monomer at the 5'-end and a LNA monomer at position 2 (calculated from the 3' end), and the antisense strand comprises a LNA monomer located at position 2 (calculated from the 3'-end). More preferably, the sense strand comprises at least one LNA monomer at the 5'-end and a LNA monomer at position 2 (calculated from the 3'-end), and the antisense strand comprises LNA monomers at position 2 and 3 (calculated from the 3'-end). Even more preferably, the sense strand comprises at least two LNA monomers at the 5'-end and LNA monomers at position 2 and 3 (calculated from the 3'-end), and the antisense strand comprises LNA monomers at position 2 and 3 (calculated from the 3'-end). Still more preferably, the sense strand comprises at least two LNA monomers at the 5'-end and LNA monomers at position 2 and 3 (calculated from the 3'-end), and the antisense strand comprises LNA monomers at position 2, 3 and 4 (calculated from the 3'-end). It will be understood that in the most preferred embodiment, none of the above-mentioned compounds contain a LNA monomer which is located at the 5'-end of the antisense strand.

As indicated above, each strand typically comprises 12-35 monomers. It will be understood that these numbers refer to the total number of naturally occurring and modified nucleotides. Thus, the total number of naturally occurring and modified nucleotides will typically not be lower than 12 and will typically not exceed 35. In an interesting embodiment of the invention, each strand comprises 17-25 monomers, such as 20-22 or 20-21 monomers.

The modified siRNA according to the invention may be blunt ended or may contain overhangs. Preferably at least one of the strands comprises a 3'-overhang. In one embodiment of the invention the sense and antisense strand both comprise a 3'-overhang. In another embodiment of the invention only the sense strand comprises a 3'-overhang.

Typically, the 3'-overhang is 1-7 monomers in length, preferably 1-5 monomers in length, such as 1-3 monomers in length, e.g. 1 monomer in length, 2 monomers in length or 3 monomers in length.

In a similar way, at least one of the strands may have a 5'-overhang. Typically, the 5'-overhang will be of 1-7 monomers in length, preferably 1-3, such as 1, 2 or 3, monomers in length. Thus, it will be understood that the sense strand may contain a 5'-overhang, the antisense strand may contain a 5'-overhang, or both of the sense and antisense strands may contain 5'-overhangs. Evidently, the sense strand may contain both a 3'- and a 5'-overhang. Alternatively, the antisense strand may contain both a 3'- and a 5'-overhang.

As far as the LNA monomers are concerned, it will be understood that any of the LNA monomers shown in Scheme 2 and 3 are useful for the purposes of the present invention. However, it is currently preferred that the LNA monomer is in the beta-D form, corresponding to the LNA monomers shown as compounds **2A, 2C** and **2D.** The currently most preferred LNA monomer is the monomer shown as compound **2A** in Schemes 2 and 3 above, i.e. the currently most preferred LNA monomer is the beta-D form of oxy-LNA.

In a further embodiment of the invention, the modified siRNA of the invention is linked to one or more ligands so as to form a conjugate. The ligand(s) serve(s) the role of increasing the cellular uptake of the conjugate relative to the non-conjugated compound. This conjugation can take place at the terminal 5'-OH and/or 3'-OH positions, but the conjugation may also take place at the sugars and/or the nucleobases. In particular, the growth factor to which the antisense oligonucleotide may be conjugated, may comprise transferrin or folate. Transferrin-polylysine-oligonucleotide complexes or folate-polylysine-oligonucleotide complexes may be prepared for uptake by cells expressing high levels of transferrin or folate receptor. Other examples of conjugates/lingands are cholesterol moieties, duplex intercalators such as acridine, poly-L-lysine, "end-capping" with one or more nuclease-resistant linkage groups such as phosphoromonothioate, and the like.

The preparation of transferrin complexes as carriers of oligonucleotide uptake into cells is described by Wagner et al, Proc. Natl. Acad. Sci. USA 87, 3410-3414 (1990). Cellular delivery of folate-macromolecule conjugates via folate receptor endocytosis, including delivery of an antisense oligonucleotide, is described by Low et al, US 5,108,921 and by Leamon et al., Proc. Natl. Acad. Sci. 88, 5572 (1991).

The compounds or conjugates of the invention may also be conjugated or further conjugated to active drug substances, for example, aspirin, ibuprofen, a sulfa drug, an antidiabetic, an antibacterial agent, a chemotherapeutic agent or an antibiotic.

### Manufacture

The modified siRNAs of the invention may be produced using the polymerisation techniques of nucleic acid chemistry, which is well known to a person of ordinary skill in the art of organic chemistry. Generally, standard oligomerisation cycles of the phosphoramidite approach (S. L. Beaucage and R. P. Iyer, Tetrahedron, 1993, 49, 6123; and S. L. Beaucage and R. P. Iyer, Tetrahedron, 1992, 48, 2223) may be used, but other chemistries, such as the H-phosphonate chemistry or the phosphortriester chemistry may also be used.

For some monomers longer coupling time and/or repeated couplings with fresh reagents and/or use of more concentrated coupling reagents may be necessary. However, in our hands, the phosphoramidites employed coupled with a satisfactory >97% step-wise coupling yield. Thiolation of the phosphate may be performed by exchanging the normal oxidation, i.e. the iodine/pyridine/H₂O oxidation, with an oxidation process using Beaucage's reagent (commercially available). As will be evident to the skilled person, other sulphurisation reagents may be employed.

Purification of the individual strands may be done using disposable reversed phase purification cartridges and/or reversed phase HPLC and/or precipitation from ethanol or butanol. Gel electrophoresis, reversed phase HPLC, MALDI-MS, and ESI-MS may be used to verify the purity of the synthesised LNA-containing oligonucleotides. Furthermore, solid support materials having immobilised thereto a nucleobase-protected and 5'-OH protected LNA are especially interesting for synthesis of the LNA-containing oligonucleotides where a LNA monomer is included at the 3' end. For this purpose, the solid support material is preferable CPG or polystyrene onto which a 3'-functionalised, optionally nucleobase protected and optionally 5'-OH protected LNA monomer is linked. The LNA monomer may be attached to the solid support using the conditions stated by the supplier for that particular solid support material.

### Therapy and Pharmaceutical Compositions

As explained initially, the modified siRNAs of the invention will constitute suitable drugs with improved properties. The design of a potent and safe RNAi drug requires the fine-tuning of various parameters such as affinity/specificity, stability in biological fluids, cellular uptake, mode of action, pharmacokinetic properties and toxicity.

Accordingly, in a further aspect the present invention relates to a pharmaceutical composition comprising a modified siRNA according to the invention and a pharmaceutically acceptable diluent, carrier or adjuvant.

In a still further aspect the present invention relates to a modified siRNA according to the invention for use as a medicament.

As will be understood dosing is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient. Optimum dosages may vary depending on the relative potency of individual siLNAs. Generally it can be estimated based on EC50s found to be effective in *in vitro* and *in vivo* animal models. In general, dosage is from 0.01 µg to 1 g per kg of body weight, and may be given once or more daily, weekly, monthly or yearly, or even once every 2 to 10 years or by continuous infusion for hours up to several months. The repetition rates for dosing can be estimated based on measured residence times and concentrations of the drug in bodily fluids or tissues. Following successful treatment, it may be desirable to have the patient undergo maintenance therapy to prevent the recurrence of the disease state.

### Pharmaceutical Composition

As indicated above the invention also relates to a pharmaceutical composition, which comprises at least one modified siRNA of the invention as an active ingredient. It should be understood that the pharmaceutical composition according to the invention optionally comprises a pharmaceutical carrier, and that the pharmaceutical composition optionally comprises further compounds, such as chemotherapeutic compounds, anti-inflammatory compounds, antiviral compounds and/or immuno-modulating compounds.

The modified siRNAs of the invention can be used "as is" or in form of a variety of pharmaceutically acceptable salts. As used herein, the term "pharmaceutically acceptable salts" refers to salts that retain the desired biological activity of the herein-identified modified siRNAs and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, *N,N*-dibenzylethylene-diamine, *D*-glucosamine, tetraethylammonium, or ethylenediamine.

In one embodiment of the invention the modified siRNA may be in the form of a pro-drug. Oligonucleotides are by virtue negatively charged ions. Due to the lipophilic nature of cell membranes the cellular uptake of oligonucleotides are reduced compared to neutral or lipophilic equivalents. This polarity "hindrance" can be avoided by using the pro-drug approach (see e.g. Crooke, R. M. (1998) in Crooke, S. T. Antisense research and Application. Springer-Verlag, Berlin, Germany, vol. 131, pp. 103-140). In this approach the oligonucleotides are prepared in a protected manner so that the oligo is neutral when it is administered. These protection groups are designed in such a way that they can be removed when the oligo is taken up by the cells. Examples of such protection groups are S-acetylthioethyl (SATE) or S-pivaloylthioethyl (*t*-butyl-SATE). These protection groups are nuclease resistant and are selectively removed intracellulary.

Pharmaceutically acceptable binding agents and adjuvants may comprise part of the formulated drug. Capsules, tablets and pills etc. may contain for example the following compounds: microcrystalline cellulose, gum or gelatin as binders; starch or lactose as excipients; stearates as lubricants; various sweetening or flavouring agents. For capsules the dosage unit may contain a liquid carrier like fatty oils. Likewise coatings of sugar or enteric agents may be part of the dosage unit. The oligonucleotide formulations may also be emulsions of the active pharmaceutical ingredients and a lipid forming a micellular emulsion. A compound of the invention may be mixed with any material that do not impair the desired action, or with material that supplement the desired action. These could include other drugs including other nucleotide compounds. For parenteral, subcutaneous, intradermal or topical administration the formulation may include a sterile diluent, buffers, regulators of tonicity and antibacterials. The active compound may be prepared with carriers that protect against degradation or immediate elimination from the body, including implants or microcapsules with controlled release properties. For intravenous administration the preferred carriers are physiological saline or phosphate buffered saline. Preferably, an oligomeric compound is included in a unit formulation such as in a pharmaceutically acceptable carrier or diluent in an amount sufficient to deliver to a patient a therapeutically effective amount without causing serious side effects in the treated patient.

The pharmaceutical compositions of the present invention may be administered in a number of ways depending upon whether local or systemic treatment is desired and upon the area to be treated. Administration may be (a) oral (b) pulmonary, e.g., by inhalation or insufflation of powders or aerosols, including by nebulizer; intratracheal, intranasal, (c) topical including epidermal, transdermal, ophthalmic and to mucous membranes including vaginal and rectal delivery; or (d) parenteral including intravenous, intraarterial, subcutaneous, intraperitoneal or intramuscular injection or infusion; or intracranial, e.g., intrathecal or intraventricular, administration. In one embodiment the pharmaceutical composition is administered IV, IP, orally, topically or as a bolus injection or administered directly in to the target organ. Pharmaceutical compositions and formulations for topical administration may include transdermal patches, ointments, lotions, creams, gels, drops, sprays, suppositories, liquids and powders. Conventional pharmaceutical carriers, aqueous, powder or oily bases, thickeners and the like may be necessary or desirable. Coated condoms, gloves and the like may also be useful. Preferred topical formulations include those in which the compounds of the invention are in admixture with a topical delivery agent such as lipids, liposomes, fatty acids, fatty acid esters, steroids, chelating agents and surfactants. Compositions and formulations for oral administration include but is not restricted to powders or granules, microparticulates, nanoparticulates, suspensions or solutions in water or non-aqueous media, capsules, gel capsules, sachets, tablets or minitablets. Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self- emulsifying solids and self-emulsifying semisolids. Delivery of drug to tumour tissue may be enhanced by carrier-mediated delivery including, but not limited to, cationic liposomes, cyclodextrins, porphyrin derivatives, branched chain dendrimers, polyethylenimine polymers, nanoparticles and microspheres (Dass CR. J Pharm Pharmacol 2002; 54(1):3-27). The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product. The compositions of the present invention may be formulated into any of many possible dosage forms such as, but not limited to, tablets, capsules, gel capsules, liquid syrups, soft gels and suppositories. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers. The compounds of the invention may also be conjugated to active drug substances, for example, aspirin, ibuprofen, a sulfa drug, an antidiabetic, an antibacterial or an antibiotic.

In another embodiment, compositions of the invention may contain one or more siLNA compounds, targeted to a first nucleic acid and one or more additional siLNA compounds targeted to a second nucleic acid target. Two or more combined compounds may be used together or sequentially.

The compounds disclosed herein are useful for a number of therapeutic applications as indicated above. In general, therapeutic methods include administration of a therapeutically effective amount of a siLNA to a mammal, particularly a human. In a certain embodiment, the present invention provides pharmaceutical compositions containing (a) one or more compounds of the invention, and (b) one or more chemotherapeutic agents. When used with the compounds of the invention, such chemotherapeutic agents may be used individually, sequentially, or in combination with one or more other such chemotherapeutic agents or in combination with radiotherapy. All chemotherapeutic agents known to a person skilled in the art are here incorporated as combination treatments with compound according to the invention. Other active agents, such as anti-inflammatory drugs, including but not limited to nonsteroidal anti-inflammatory drugs and corticosteroids, antiviral drugs, and immuno-modulating drugs may also be combined in compositions of the invention. Two or more combined compounds may be used together or sequentially.

### Cancer

In an even further aspect the present invention relates to the use of a modified siRNA according to the invention for the manufacture of a medicament for the treatment of cancer. In another aspect the modified siRNAs of the invention may be used in a method for treatment of, or prophylaxis against, cancer, said method comprising administering a modified siRNA of the invention or a pharmaceutical composition of the invention to a patient in need thereof.

Such cancers may include lymphoreticular neoplasia, lymphoblastic leukemia, brain tumors, gastric tumors, plasmacytomas, multiple myeloma, leukemia, connective tissue tumors, lymphomas, and solid tumors.

In the use of a compound of the invention for the manufacture of a medicament for the treatment of cancer, said cancer may suitably be in the form of a solid tumor. Analogously, in the method for treating cancer disclosed herein said cancer may suitably be in the form of a solid tumor.

Furthermore, said cancer is also suitably a carcinoma. The carcinoma is typically selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumors. More typically, said carcinoma is selected from the group consisting of malignant melanoma, non-small cell lung cancer, breast carcinoma, colon carcinoma and renal cell carcinoma. The malignant melanoma is typically selected from the group consisting of superficial spreading melanoma, nodular melanoma, lentigo maligna melanoma, acral melagnoma, amelanotic melanoma and desmoplastic melanoma.

Alternatively, the cancer may suitably be a sarcoma. The sarcoma is typically in the form selected from the group consisting of osteosarcoma, Ewing's sarcoma, chondrosarcoma, malignant fibrous histiocytoma, fibrosarcoma and Kaposi's sarcoma.

Alternatively, the cancer may suitably be a glioma.

A further embodiment is directed to the use of a modified siRNA according to the invention for the manufacture of a medicament for the treatment of cancer, wherein said medicament further comprises a chemotherapeutic agent selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine). Suitably, the further chemotherapeutic agent is selected from taxanes such as Taxol, Paclitaxel or Docetaxel.

Similarly, the invention is further directed to the use of a modified siRNA according to the invention for the manufacture of a medicament for the treatment of cancer, wherein said treatment further comprises the administration of a further chemotherapeutic agent selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine). Suitably, said treatment further comprises the administration of a further chemotherapeutic agent selected from taxanes, such as Taxol, Paclitaxel or Docetaxel.

Alternatively stated, the modified siRNA of the invention may be used in a method for treating cancer, said method comprising administering a modified siRNA of the invention or a pharmaceutical composition according to the invention to a patient in need thereof and further comprising the administration of a further chemotherapeutic agent. Said further administration may be such that the further chemotherapeutic agent is conjugated to the compound of the invention, is present in the pharmaceutical composition, or is administered in a separate formulation.

### Infectious Diseases

In a particular interesting embodiment of the invention, the modified siLNA compounds according to the invention are used for targeting Severe Acute Respiratory Syndrome (SARS), which first appeared in China in November 2002. According to the WHO over 8,000 people have been infected world-wide, resulting in over 900 deaths. A previously unknown coronavirus has been identified as the causative agent for the SARS epidemic (Drosten C et al. N Engl J Med 2003,348,1967-76; and Fouchier RA et al. Nature 2003,423,240). Identification of the SARS-CoV was followed by rapid sequencing of the viral genome of multiple isolates (Ruan et al. Lancet 2003,361,1779-85; Rota PA et al. Science 2003,300,1394-9; and Marra MA et al. Science 2003,300,399-404). This sequence information immediately made possible the development of SARS antivirals by nucleic acid-based knock-down techniques such as siRNA. The nucleotide sequence encoding the SARS-CoV RNA-dependent RNA polymerase (Pol) is highly conserved throughout the coronavirus family. The Pol gene product is translated from the genomic RNA as a part of a polyprotein, and uses the genomic RNA as a template to synthesize negative-stranded RNA and subsequently sub-genomic mRNA. The Pol protein is thus expressed early in the viral life cycle and is crucial to viral replication.

Accordingly, in a further another aspect the present invention relates the use of a modified siRNA according to the invention for the manufacture of a medicament for the treatment of Severe Acute Respiratory Syndrome (SARS). The modified siRNAs of the invention may be used in a method for treating Severe Acute Respiratory Syndrome (SARS), said method comprising administering a modified siRNA according to the invention or a pharmaceutical composition according to the invention to a patient in need thereof.

It is contemplated that the compounds of the invention may be broadly applicable to a broad range of infectious diseases, such as diphtheria, tetanus, pertussis, polio, hepatitis B, hemophilus influenza, measles, mumps, and rubella.

Accordingly, in yet another aspect the present invention relates the use of a modified siRNA according to the invention for the manufacture of a medicament for the treatment of an infectious disease. The modified siRNAs of the invention may be used in a method for treating an infectious disease, said method comprising administering a modified siRNA according to the invention or a pharmaceutical composition according to the invention to a patient in need thereof.

### Inflammatory Diseases

The inflammatory response is an essential mechanism of defense of the organism against the attack of infectious agents, and it is also implicated in the pathogenesis of many acute and chronic diseases, including autoimmune disorders. In spite of being needed to fight pathogens, the effects of an inflammatory burst can be devastating. It is therefore often necessary to restrict the symptomatology of inflammation with the use of anti-inflammatory drugs. Inflammation is a complex process normally triggered by tissue injury that includes activation of a large array of enzymes, the increase in vascular permeability and extravasation of blood fluids, cell migration and release of chemical mediators, all aimed to both destroy and repair the injured tissue.

In yet another aspect, the present invention relates to the use of a modified siRNA according to the invention for the manufacture of a medicament for the treatment of an inflammatory disease. The modified siRNAs of the invention may be used in a method for treating an inflammatory disease, said method comprising administering a modified siRNA according to the invention or a pharmaceutical composition according to the invention to a patient in need thereof.

In one preferred embodiment of the invention, the inflammatory disease is a rheumatic disease and/or a connective tissue diseases, such as rheumatoid arthritis, systemic lupus erythematous (SLE) or Lupus, scleroderma, polymyositis, inflammatory bowel disease, dermatomyositis, ulcerative colitis, Crohn's disease, vasculitis, psoriatic arthritis, exfoliative psoriatic dermatitis, pemphigus vulgaris and Sjorgren's syndrome, in particular inflammatory bowel disease and Crohn's disease.

Alternatively, the inflammatory disease may be a non-rheumatic inflammation, like bursitis, synovitis, capsulitis, tendinitis and/or other inflammatory lesions of traumatic and/or sportive origin.

### Other Uses

The modified siRNAs of the present invention can be utilized for as research reagents for diagnostics, therapeutics and prophylaxis. In research, the modified siRNA may be used to specifically inhibit the synthesis of target genes in cells and experimental animals thereby facilitating functional analysis of the target or an appraisal of its usefulness as a target for therapeutic intervention. In diagnostics the siRNA oligonucleotides may be used to detect and quantitate target expression in cell and tissues by Northern blotting, *in-situ* hybridisation or similar techniques. For therapeutics, an animal or a human, suspected of having a disease or disorder, which can be treated by modulating the expression of target is treated by administering the modified siRNA compounds in accordance with this invention. Further provided are methods of treating an animal particular mouse and rat and treating a human, suspected of having or being prone to a disease or condition, associated with expression of target by administering a therapeutically or prophylactically effective amount of one or more of the modified siRNA compounds or compositions of the invention.

The invention is further illustrated in a non-limiting manner by the following examples.

### EXAMPLES

### Abbreviations

- DMT:: Dimethoxytrityl
- DCI:: 4,5-Dicyanoimidazole
- DMAP:: 4-Dimethylaminopyridine
- DCM:: Dichloromethane
- DMF:: Dimethylformamide
- THF:: Tetrahydrofuran
- DIEA:: *N,N*-diisopropylethylamine
- PyBOP:: Benzotriazole-1-yl-oxy-tris-pyrrolidino-phosphonium hexafluorophosphate
- Bz:: Benzoyl
- Ibu:: Isobutyryl
- Beaucage:: 3H-1,2-Benzodithiole-3-one-1,1-dioxide
- GL3+:: 5'-cuuacgcugaguacuucga_{d}t_{d}t-3',
- GL3-:: 5'-ucgaaguacucagcguaag_{d}t_{d}t-3'
- NPY+:: 5'-ugagagaaagcacagaaaa_{d}t_{d}t-3'
- NPY-:: 5'-uuuucugugcuuucucuca_{d}t_{d}t-3'
- RL+:: 5'-aucugaagaaggagaaaaa_{d}t_{d}t-3'
- RL-:: 5'-uuuuucuccuucuucagau_{d}t_{d}t-3'
- 2187:: 5'-TcgaaguacucagcguaagTT-3'
- 2789:: 5'-u^{Me}CgaaguacucagcguaagTT-3'
- 2790:: 5'-ucGaaguacucagcguaagTT-3'
- 2792:: 5'-ucgaAguacucagcguaagTT-3'
- 2793:: 5'-ucgaaGuacucagcguaagTf-3'
- 2794:: 5'-ucgaaguAcucagcguaagTT-3'
- 2864:: 5'-ucgaaguaMeCucagcguaagTT-3'
- 2865:: 5'-ucgaaguacTcagcguaagTT-3'
- 2865-U:: 5'-ucgaaguacAcagcguaagTT-3'
- 2866:: 5'-ucgaaguacu^{Me}CagcguaagTT-3'
- 2867:: 5'-ucgaaguacucAgcguaagTT-3'
- Small letters without prefix:: RNA monomer
- Small letters with "d" prefix:: DNA monomer
- Capital letters:: Beta-D-oxy LNA monomer

### Example 1: Monomer Synthesis

The preparation of LNA monomers is described in great detail in the references Koshkin et al., J. Org. Chem., 2001,66,8504-8512, and Pedersen et al., Synthesis, 2002,6,802-809 as well as in references given therein. Where the Z and Z* protection groups were oxy-N,N-diisopropyl-O-(2-cyanoethyl)phosphoramidite and dimethoxytrityloxy such compounds were synthesised as described in WO 03/095467; Pedersen et al., Synthesis 6, 802-808, 2002; S⌀rensen et al., J. Am. Chem. Soc., 124, 2164-2176, 2002; Singh et al., J. Org. Chem. 63, 6078-6079, 1998; and Rosenbohm et al., Org. Biomol. Chem. 1, 655-663, 2003. All cytosine-containing monomers were replaced with 5-methyl-cytosine monomers for all couplings. All LNA monomers used were beta-D-oxy LNA (compound 2A).

### Example 2: Oligonucleotide Syntheses

All syntheses were carried out in 1 µmole scale on a MOSS Expedite instrument platform. The synthesis procedures were carried out essentially as described in the instrument manual.

### Preparation of LNA Succinyl Hemiester

5'-O-DMT-3"hydroxy-LNA monomer (500 mg), succinic anhydride (1.2 eq.) and DMAP (1.2 eq.) were dissolved in DCM (35 ml). The reaction mixture was stirred at room temperature overnight. After extraction with NaH₂PO₄, 0.1 M, pH 5.5 (2x), and brine (1x), the organic layer was further dried with anhydrous Na₂SO₄, filtered, and evaporated. The hemiester derivative was obtained in a 95% yield and was used without any further purification.

### Preparation of LNA-CPG (Controlled Pore Glass)

The above-prepared hemiester derivative (90 µmole) was dissolved in a minimum amount of DMF. DIEA and pyBOP (90 µmole) were added and mixed together for 1 min. This preactivated mixture was combined with LCAA-CPG (500 Å, 80-120 mesh size, 300 mg) in a manual synthesiser and stirred. After 1.5 h stirring at room temperature, the support was filtered off and washed with DMF, DCM and MeOH. After drying the loading was determined to be 57 µmol/g (see Tom Brown, Dorcas J.S.Brown. Modern machine-aided methods of oligodeoxyribonucleotide synthesis. In: F.Eckstein, editor. Oligonucleotides and Analogues A Practical Approach. Oxford: IRL Press, 1991: 13-14).

### Phosphorothioate Cycles

5'-O-DMT (A(bz), C(bz), G(ibu) or T) linked to CPG were deprotected using a solution of 3% trichloroacetic acid (v/v) in dichloromethane. The CPG was washed with acetonitrile. Coupling of phosphoramidites (A(bz), G(ibu), 5-methyl-C(bz)) or T -β-cyanoethyl-phosphoramidite) was performed by using 0.08 M solution of the 5'-O-DMT-protected amidite in acetonitrile and activation was done by using DCI (4,5-dicyanoimidazole) in acetonitrile (0.25 M). The coupling reaction was carried out for 2 min. Thiolation was carried out by using Beaucage reagent (0.05 M in acetonitrile) and was allowed to react for 3 min. The support was thoroughly washed with acetonitrile and the subsequent capping was carried out by using standard solutions (CAP A) and (CAP B) to cap unreacted 5' hydroxyl groups. The capping step was then repeated and the cycle was concluded by acetonitrile washing.

### LNA Unit Cycles

5'-O-DMT (A(bz), C(bz), G(ibu) or T) linked to CPG was deprotected by using the same procedure as described above. Coupling was performed by using 5'-O-DMT-A(bz), C(bz), G(ibu) or T-β-cyanoethylphosphoramidite (0.1 M in acetonitrile) and activation was done by DCI (0.25 M in acetonitrile). The coupling reaction was carried out for 7 minutes. Capping was done by using standard solutions (CAP A) and (CAP B) for 30 sec. The phosphite triester was oxidized to the more stable phosphate triester by using a standard solution of I₂ and pyridine in THF for 30 sec. The support was washed with acetonitrile and the capping step was repeated. The cycle was concluded by thorough acetonitrile wash.

### Cleavage and Deprotection

The oligonucleotides were cleaved from the support and the β-cyanoethyl protecting group removed by treating the support with 35% NH₄OH for 1 h at room temperature. The support was filtered off and the base protecting groups were removed by raising the temperature to 65°C for 4 hours. Ammonia was then removed by evaporation.

### Purification

The oligos were either purified by reversed-phase-HPLC (RP-HPLC) or by anion exchange chromatography (AIE):
*RP-HPLC:*

| | |
|---|---|
| Column: | VYDAC^{™}, Cat. No. 218TP1010 (vydac) |
| Flow rate: | 3 ml/min |
| Buffer: | A (0.1 M ammonium acetate, pH 7.6) |
| | B (acetonitrile) |

*Gradient:*

| | | | | | | |
|---|---|---|---|---|---|---|
| Time | 0 | 10 | 18 | 22 | 23 | 28 |
| B% | 0 | 5 | 30 | 100 | 100 | 0 |

*AIE:*

| | |
|---|---|
| Column: | Resource^{™} 15Q (amersham pharmacia biotech) |
| Flow rate: | 1.2 ml/min |
| Buffer: | A (0.1 M NaOH) |
| | B (0.1 M NaOH, 2.0 M NaCl) |

*Gradient:*

| | | | | | | |
|---|---|---|---|---|---|---|
| Time | 0 | 1 | 27 | 28 | 32 | 33 |
| B% | 0 | 25 | 55 | 100 | 100 | 0 |

### Tₘ Measurements

Melting curves were recorded on a Perkin Elmer UV/VIS spectrophotometer lambda 40 attached to a PTP-6 Peltier System. Oligonucleotides were dissolved in salt buffer (10 mM phosphate buffer, 100 mM NaCl, 0,1 mM EDTA, pH 7.0) at a concentration of 1.5 µM and using 1 cm path-length cells. Samples were denatured at 95°C for 3 min and slowly cooled to 20°C prior to measurements. Melting curves were recorded at 260 nm using a heating rate of 1°C/min, a slit of 2 nm and a response of 0.2 sec. Tm values were obtained from the maximum of the first derivative of the melting curves.

### Example 3: Synthesis of LNA/RNA Oligonucleotides

### Synthesis

LNA/RNA oligonucleotides were synthesized DMT-off on a 1.0 µmole scale using an automated nucleic acid synthesiser (MOSS Expedite 8909) and using standard reagents. 1*H*-tetrazole or 5-ethylthio-1*H*-tetrazole were used as activators. The LNA A^{Bz} , G^{lBu} and T phosphoramidite concentration was 0.1 M in anhydrous acetonitrile. The ^{Me}C^{Bz} was dissolved in 15 % THF in acetonitrile. The coupling time for all monomer couplings was 600 secs. The RNA phosphoramidites (Glen Research, Sterling, Virginia) were N-acetyl and 2'-O-triisopropylsilyloxymethyl (TOM) protected. The monomer concentration was 0.1 M (anhydrous acetonitrile) and the coupling time was 900 secs. The oxidation time was set to be 50 sec. The solid support was DMT-LNA-CPG (1000 Å, 30-40 µmole/g).

### Work-up and Purification

Cleavage from the resin and nucleobase/phosphate deprotection was carried out in a sterile tube by treatment with 1.5 ml of a methylamine solution (1:1, 33% methylamine in ethanol:40% methylamine in water) at 35°C for 6 h or left overnight. The tube was centrifuged and the methylamine solution was transferred to second sterile tube. The methylamine solution was evaporated in a vacuum centrifuge. To remove the 2'-O-protection groups the residue was dissolved in 1.0 ml 1.0 M TBAF in THF and heated to 55°C for 15 min. and left at 35°C overnight. The THF was evaporated in a vacuum centrifuge leaving a light yellow gum, which was neutralised with approx. 600 µl (total sample volume: 1.0 ml) of RNase-free 1.0 M Tris-buffer (pH 7). The mixture was homogenised by shaking and heating to 65°C for 3 min. Desalting of the oligonucleotides was performed on NAP-10 columns (Amersham Biosciences, see below). The filtrate from step 4 (see below) was collected and analysed by MALDI-TOF and gel electroforesis (16% sequencing acrylamide gel (1 mm), 0.9% TBE [Tris: 89 mM, Boric acid: 89 mM, EDTA: 2 mM, pH 8.3] buffer, ran for 2 h at 20 W as the limiting parameter. The gel was stained in CyberGold (Molecular Probes, 1:10000 in 0.9xTBE) for 30 min followed by scanning in a Bio-Rad FX Imager). The concentration of the oligonucleotide was measured by UV-spectrometry at 260 nm.

### Scheme A, Desalting on NAP-10 columns:

| Step | Reagent | Operation | Volume | Remarks |
|---|---|---|---|---|
| 1 | - | Empty storage buffer | - | Discard |
| 2 | H₂O (RNase-free) | Wash | 2 x full volume | Discard |
| 3 | Oligo in buffer (RNase-free) | Load | 1.0 ml | Discard |
| 4 | H₂O (RNase-free) | Elution | 1.5 ml | Collect - Contains oligo |
| 5 | H₂O (RNase-free) | "Elution" | 0.5 ml | Collect - Contains salt + small amount of oligo |

As will be appreciated by the skilled person, the most important issues in the synthesis of the LNA/RNA oligos as compared to standard procedures are that i) extended coupling times are necessary to achieve good coupling efficiency, and ii) the oxidation time has to be extended to minimise the formation of deletion fragments. Furthermore, coupling of 2'-*O*-TOM protected phosphoramidites were superior to 2'-*O*-TBDMS. Taking this into account, the crude oligonucleotides were of such quality that further purification could be avoided. MS analysis should be carried out after the TOM-groups are removed.

### Example 4: Test of Design of modified siRNA in Mammalian Reporter System

The efficacy of different modified siRNA designs and combinations were first assessed in a luciferase reported system in mammalian cell culture. Sense and the corresponding antisense oligonucleotides were hybridised to generate double strands.

The cells used were the human embryonal kidney (HEK) 293 cell lines. HEK 293 cells were maintained in DMEM supplemented with 10% foetal bovine serum, penicillin, strepto-mycine and glutamine (Invitrogen, Paisely, UK). The plasmids used were pGL3-Control coding for firefly luciferace under the control of the SV40 promoter and enhancer and pRL-TK coding for *Renilla* luciferase under the control of HSV-TK promoter (Promega, Madison, WI, USA).

### Transfection

One day before transfection cells were seeded in 500 µl medium in 24-well plates in order to adhere and reach a confluency of 70 to 90% at the time of transfection. Cells were seeded in the medium without antibiotics and changed to 500 µl Opti-MEM I just before adding the transfection mix to the cells. A standard co-transfection mix was prepared for triplicate wells by separately adding 510 ng pGL3-Control, 51 ng pRL-TK and 340 ng siRNA to 150 µl Opti-MEM I (Invitrogen) and 3 µl LipofectAMINE 2000 (Invitrogen) to another 150 µl Opti-MEM I. The two solutions were mixed and incubated at room temperature for 20-30 minutes before adding to the cells. 100 µl of the transfection mix was added to each of the three wells. The final volume of medium plus transfection mix was 600 µl. The siLNA or siRNA concentration corresponded to about 13 nM. Cells were incubated with the transfection mix for 4 hours and the medium was then changed with fully supplemented DMEM.

### Dual-Luciferase Reporter Assay (Promega)

Cells were harvested in passive lysis buffer and assayed according to the protocol (Promega) using a NovoSTAR 96-well format luminometer with substrate dispenser (BMG Labtechnologies, Offenburg, Germany). 10 µl sample was applied in each well of a 96 well plate and 50 µl Luciferace Assay Reagent II (substrate for firefly luciferase) was added to a well by the luminometer and measured. Then, 50 µl Stop and Glow (stop solution for firefly luciferase and substrate for *Renilla* luciferase) was added and measured. The average of the luciferase activities measured for 10 sec. was used to calculate ratios between firefly and *Renilla* luciferase or the opposite.

## Claims

1. A modified siRNA comprising a sense strand and an antisense strand, wherein the sense strand comprises a modified RNA nucleotide in at least one of positions 8-14, calculated from the 5'-end, and wherein the sense strand comprises a non-RNA nucleobase in position 10, calculated from the 5'-end, wherein the non-RNA nucleobase has a sugar moiety which differs from ribose and wherein the modified siRNA is able to specifically interfer with protein expression.

2. The modified siRNA according to claim 1, wherein the sense strand also comprises a non-RNA nucleobase in position 12, calculated from the 5'-end.

3. The modified siRNA according to claim 1, wherein the sense strand also comprises a non-RNA nucleobase in position 11, calculated from the 5'-end.

4. The modified siRNA according to any of claims 1-3, wherein said non-RNA nucleobase is selected from the group consisting of thymine (T), 5-methylcytosine (^{Me}C), isocytosine, pseudoisocytosine, 5-bromouracil, 5-propynyluracil, 5-propyny-6-fluoroluracil, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine.

5. The modified siRNA according to claim 4, wherein said non-RNA nucleobase is T or ^{Me}C.

6. The modified siRNA according to claim 5, wherein said non-RNA nucleobase is T.

7. The modified siRNA according to any of claims 1-6, wherein said sugar moiety differs from ribose in that the ribose 2'-OH group has been modified.

8. The modified siRNA according to claim 7, wherein said sugar moiety is LNA.

9. The modified siRNA according to claim 8, wherein said LNA is selected from the group consisting of thio-LNA, amino-LNA, oxy-LNA and ena-LNA.

10. The modified siRNA according to claim 9, wherein said LNA is oxy-LNA.

11. The modified siRNA according to claim 10, wherein said oxy-LNA is in the beta-D form.

12. The modified siRNA according to any of the preceding claims, wherein the sense strand comprises a sugar moiety which differs from ribose in position 12, calculated from the 5'-end, and wherein the sense strand comprises a non-RNA nucleobase in position 10, calculated from the 5'-end.

13. The modified siRNA according to any of the preceding claims, wherein each strand comprises 12-35 monomers.

14. A pharmaceutical composition comprising the modified siRNA as defined in any of claims 1-13 and a pharmaceutically acceptable diluent, carrier or adjuvant.

15. The modified siRNA according to any of claims 1-13 for use as a medicament.

## Patentansprüche

1. Modifizierte siRNA, umfassend einen sense-Strang und einen antisense-Strang, wobei der sense-Strang in mindestens einer der Positionen 8-14, gezählt von dem 5'-Ende, ein modifiziertes RNA-Nukleotid umfasst und wobei der sense-Strang in Position 10, gezählt von dem 5'-Ende, eine Nicht-RNA-Nukleobase umfasst, wobei die Nicht-RNA-Nukleobase eine von Ribose verschiedene Zuckergruppe aufweist und wobei die modifizierte siRNA spezifisch die Proteinexpression stören kann.

2. Modifizierte siRNA nach Anspruch 1, wobei der sense-Strang weiterhin eine Nicht-RNA-Nukleobase in Position 12, gezählt von dem 5'-Ende, umfasst.

3. Modifizierte siRNA nach Anspruch 1, wobei der sense-Strang weiterhin eine Nicht-RNA-Nukleobase in Position 11, gezählt von dem 5'-Ende, umfasst.

4. Modifizierte siRNA nach einem der Ansprüche 1-3, wobei die Nicht-RNA-Nukleobase ausgewählt ist aus der Gruppe, bestehend aus Thymin (T), 5-Methylcytosin (^{Me}C), Isocytosin, Pseudoisocytosin, 5-Bromouracil, 5-Propinyluracil, 5-Propinyl-6-fluorouracil, 5-Methylthiazoluracil, 6-Aminopurin, 2-Aminopurin, Inosin, 2,6-Diaminopurin, 7-Propinyl-7-deazaadenin, 7-Propinyl-7-deazaguanin und 2-Chlor-6-aminopurin.

5. Modifizierte siRNA nach Anspruch 4, wobei die Nicht-RNA-Nukleobase T oder ^{Me}C ist.

6. Modifizierte siRNA nach Anspruch 5, wobei die Nicht-RNA-Nukleobase T ist.

7. Modifizierte siRNA nach einem der Ansprüche 1-6, wobei sich die Zuckergruppe dadurch von Ribose unterscheidet, dass die 2'-OH-Gruppe von Ribose modifiziert ist.

8. Modifizierte siRNA nach Anspruch 7, wobei die Zuckergruppe LNA ist.

9. Modifizierte siRNA nach Anspruch 8, wobei die LNA ausgewählt ist aus der Gruppe, bestehend aus Thio-LNA, Amino-LNA, Oxy-LNA und Ena-LNA.

10. Modifizierte siRNA nach Anspruch 9, wobei die LNA Oxy-LNA ist.

11. Modifizierte siRNA nach Anspruch 10, wobei die Oxy-LNA in der Beta-D-Form vorliegt.

12. Modifizierte siRNA nach einem der vorhergehenden Ansprüche, wobei der sense-Strang in Position 12, gezählt von dem 5'-Ende, eine von Ribose verschiedene Zuckergruppe umfasst und wobei der sense-Strang in Position 10, gezählt von dem 5'-Ende, eine Nicht-RNA-Nukleobase umfasst.

13. Modifizierte siRNA nach einem der vorhergehenden Ansprüche, wobei jeder Strang 12-35 Monomere umfasst.

14. Pharmazeutische Zusammensetzung, umfassend die modifizierte siRNA nach einem der Ansprüche 1-13 und ein pharmazeutisch unbedenkliches Verdünnungsmittel, Träger oder Adjuvans.

15. Modifizierte siRNA nach einem der Ansprüche 1-13 zur Verwendung als Arzneimittel.

## Revendications

1. Petit ARN interférent modifié comprenant un brin sens et un brin antisens, dans lequel le brin sens comprend un nucléotide d'ARN modifié en au moins une des positions 8 à 14, calculée depuis l'extrémité 5', et dans lequel le brin sens comprend une nucléobase « non-ARN » en position 10, calculée depuis l'extrémité 5', dans lequel la nucléobase « non-ARN » possède une fraction sucre qui diffère du ribose et dans lequel le petit ARN interférent modifié est capable d'interférer de manière spécifique avec l'expression des protéines.

2. Le petit ARN interférent modifié selon la revendication 1, dans lequel le brin sens comprend aussi une nucléobase « non-ARN » en position 12, calculée depuis l'extrémité 5'.

3. Le petit ARN interférent modifié selon la revendication 1, dans lequel le brin sens comprend aussi une nucléobase « non-ARN » en position 11, calculée depuis l'extrémité 5'.

4. Le petit ARN interférent modifié selon l'une quelconque des revendications 1 à 3, dans lequel ladite nucléobase « non-ARN » est choisie dans le groupe consistant en thymine (T), 5-méthylcytosine (^{Me}C), isocytosine, pseudoisocytosine, 5-bromouracile, 5-propynyluracile, 5-propyny-6-fluoroluracile, 5-méthylthiazoleuracile, 6-aminopurine, 2-aminopurine, inosine, 2,6-diaminopurine, 7-propyne-7-déazaadénine, 7-propyne-7-déazaguanine et 2-chloro-6-aminopurine.

5. Le petit ARN interférent modifié selon la revendication 4, dans lequel ladite nucléobase « non-ARN » est T ou ^{Me}C.

6. Le petit ARN interférent modifié selon la revendication 5, dans lequel ladite nucléobase « non-ARN » est T.

7. Le petit ARN interférent modifié selon l'une quelconque des revendications 1 à 6, dans lequel ladite fraction sucre diffère du ribose en ce que le groupe 2'-OH du ribose a été modifié.

8. Le petit ARN interférent modifié selon la revendication 7, dans lequel ladite fraction sucre est un LNA.

9. Le petit ARN interférent modifié selon la revendication 8, dans lequel ledit LNA est choisi dans le groupe consistant en thio-LNA, amino-LNA, oxy-LNA et ena-LNA.

10. Le petit ARN interférent modifié selon la revendication 9, dans lequel ledit LNA est un oxy-LNA.

11. Le petit ARN interférent modifié selon la revendication 10, dans lequel ledit oxy-LNA est sous la forme bêta-D.

12. Le petit ARN interférent modifié selon l'une quelconque des revendications précédentes, dans lequel le brin sens comprend une fraction sucre qui diffère du ribose en position 12, calculée depuis l'extrémité 5', et dans lequel le brin sens comprend une nucléobase « non-ARN » en position 10, calculée depuis l'extrémité 5'.

13. Le petit ARN interférent modifié selon l'une quelconque des revendications précédentes, dans lequel chaque brin comprend 12 à 35 monomères.

14. Composition pharmaceutique comprenant le petit ARN interférent modifié tel que défini dans l'une quelconque des revendications 1 à 13 et un solvant, un véhicule ou un adjuvant pharmaceutiquement acceptable.

15. Le petit ARN interférent modifié selon l'une quelconque des revendications 1 à 13 destiné à être utilisé comme médicament.
